# EUROPÄISCHE PATENTANMELDUNG

(11) **EP 3 524 055 A1**
(43) Veröffentlichungstag der Anmeldung: **14.08.2019**
(21) Anmeldenummer: 18155747.1
(22) Anmeldetag: 08.02.2018
(51) Int. Cl.: A01N 47/44, A61K 9/00, A61K 31/09, A61K 31/167, A61K 31/785

(54) **ANTIBAKTERIELLES UND SPERMIZIDES GLEITMITTEL**

(71) Anmelder: BCSK Biocid GmbH, 1010 Wien (AT)
(72) Erfinder: Lukas, MAYERHOFER, 1040 Wien (AT)
(74) Vertreter: Sonn & Partner Patentanwälte

(57) **Zusammenfassung**

Die Erfindung betrifft eine chemische Zusammensetzung enthaltend mindestens ein polymeres Guanidin Biozid und mindestens ein Alkylphenoxypolyethoxyethanol Spermizid, in wässriger Lösung, und mindestens ein Verdickungsmittel, welche als biozides und spermizides Mittel zur Anwendung während eines Geschlechtsaktes geeignet ist.

## Beschreibung

Die vorliegende Erfindung betrifft eine Intimanwendung mit einem Spermizid in einer stabilen Gelformulierung.

Intimformulierungen mit einem Spermizid sind in der Literatur beschrieben. So beschreibt etwa die US 5,512,289 eine Formulierung mit einem Alkylphenoxypolyethoxyethanol Spermizid, wie z.B. das übliche Spermizid Nonoxynol-9, und einem Lösungsvermittler aus der Gruppe der polyethoxylierten nichtionischen Verbindungen, vordergründig Polyethylenglykol (PEG) oder Poylsorbat (Tween). Hohe Mengen derartiger Lösungsvermittler v.a. von PEG, sind essentiell, da ansonsten das Spermizid ausfällt und milchige Pasten entstehen. PEG und Poylsorbat sind bedenkliche Stoffe, da sie die Barrierefunktion der Haut senken. Dies ist insbesondere bei einer Intimformulierung, die zudem das Infektionsrisiko senken soll, ungeeignet.

Alkylphenoxypolyethoxyethanole, insbesondere Nonoxynol-9, sind selbst starke oberflächenaktive Substanzen (US 6,028,115) und es ist daher für ihre Wirkung abträglich, wenn sich in einer Formulierung Mikrophasentrennungen, wie Emulsionen, bilden, da hierbei Mikroheterogenitäten in der Formulierung entstehen, welche Zonen unterschiedliche, inklusive stark abgeschwächte, Aktivitäten, bilden.

Es ist eine Aufgabe der vorliegenden Erfindungen alternative Formulierungen mit einem Alkylphenoxypolyethoxyethanol Spermizid bereitzustellen, die auf die Zugabe von polymeren Verbindungen wie PEG oder Polysorbat verzichten können. Eine weitere Aufgabe liegt in der Bereitstellung eines bioziden Wirkstoffes in einem Gleitmittel um die Gefahr der Ansteckung mit einer Geschlechtskrankheit zu reduzieren.

Beide Aufgaben wurden erfindungsgemäß mit der Verwendung von polymeren Guanidinen als biozide Substanzen erzielt, welche zudem überraschenderweise stabile verdickte Lösungen in Gleitgelen ermöglichen. Stabil bedeutet hierin, dass eine Phasentrennung, wie in den Vergleichsbeispielen der US 5,512,289 beobachtet, vermieden wird.

Überraschenderweise hat sich herausgestellt, dass auf PEG, Polysorbat oder andere Lösungsvermittler der Gruppe der polyethoxylierten nichtionischen Verbindungen verzichtet werden kann, wenn der Formulierung der antimikrobielle Wirkstoff aus der Gruppe der polymeren Guanidine in geringen Mengen beigefügt wird.

Die Erfindung betrifft daher eine chemische Zusammensetzung enthaltend mindestens ein polymeres Guanidin Biozid und mindestens ein Alkylphenoxypolyethoxyethanol Spermizid, in wässriger Lösung, und mindestens ein Verdickungsmittel.

Die Zusammensetzung ermöglicht die Unterbindung der Übertragung von Geschlechtserkrankungen während eines Sexualakts und der gleichzeitigen Empfängnisverhütung ohne die Verwendung eines mechanischen Schutzes. Die Verwendung einer Gelformulierung bietet den Vorteil eines Gleitgels um einen schmerzfreien und reibungsreduzierten Geschlechtsverkehr zu ermöglichen. Dadurch unterbleiben die während eines Geschlechtsakts ohne Gleitmittel auftretenden Mikroläsionen, die ein zusätzliches Infektionsrisiko darstellen. Hierbei hat sich überraschenderweise herausgestellt, dass durch die erfindungsgemäße Zusammensetzung die biozide Wirkung des polymeren Guanidin Biozids verstärkt.

"Unterbindung" oder auch "Verhinderung" sollen nicht als absolute Effekte verstanden werden, i.e. als eine Verhinderung mit 100%iger Erfolgswahrscheinlichkeit, sondern als eine Reduzierung der Gefahr oder Wahrscheinlichkeit einer Übertragung von Geschlechtskrankheiten und/oder einer Empfängnis.

Polymere Guanidin Biozide sind im Fachgebiet der Desinfektionsmittel bekannt. Ihre Herstellung ist in der WO 01/85676 A1 und in den Patenten AT 408302 B und AT 411060 B beschrieben. Polymere Guanidin Biozide werden auch Akacid, oder X-Cid, bezeichnet und sind in Kratzer et al., Antibiotika Monitor 1/2/2006; Buxbaum et al., Journal of Antimicrobial Chemotherapy (2006) 58, 193-197; US 2,325,586; GB 1095902 A; WO 1999/054291 A1; WO 01/85676 A1; WO 2002/030877 A1; WO 2006/047800 A1; WO 2008/080184 A2; WO 2009/092123 A2; EP 2520605 A1; WO 2013/064161 A1; WO 2014/113835 A1; WO 2016/015081 A1 beschrieben (alle durch Bezugnahme hierin aufgenommen). Das polymere Guanin Biozid kann auch als Komplex, z.B. mit Gelatine oder einem Polysaccharid, vorliegen (z.B. wie in der WO 2010/106007 A1 beschrieben). Die WO 2008/080184 beschreibt die Verwendung von polymeren Guanidinen zur Bekämpfung von Mikroorganismen bei nichttherapeutischen Anwendungen, z.B. durch Vernebelung zur Raumdesinfektion. Eine darin genannte Zusammensetzung hierzu ist Akacid, Poly-[2-(2-ethoxy)-ethoxyethyl-guanidinium-chlorid und Akacid plus, eine 3:1-Mischung aus Poly(hexamethylenguanidiniumchlorid) und Poly[2-(2-ethoxy)ethoxyethyl)-guanidiniumchlorid].

Hierin wird der Begriff "polymere Guanidine" insbesondere für "polymere Guanidinderivate auf Basis eines Alkylendiamins und/oder eines Oxyalkylendiamins" (WO 2009/009815 A1), speziell für "biozide polymere Guanidinderivate auf der Basis von Diaminen, welche Alkylketten oder Oxyalkylenketten zwischen zwei Aminogruppen enthalten, wobei die Guanidinderivate ein Produkt der Polykondensation eines Guanidin-Säureadditionssalzes mit Diaminen, welche Polyalkylenketten oder Polyoxyalkylenketten zwischen zwei Aminogruppen enthalten, darstellen" (EP 1 280 766 B1, Anspruch 1), verwendet. Besonders bevorzugt sind ein Poly(hexamethylenguanidinium) Salz und/oder Poly[2-(2-ethoxy)ethoxyethyl)-guanidinium Salz. Akacid und Akacid Plus sind bevorzugte polymere Guanidin Biozide gemäß der vorliegenden Erfindung.

Eine bevorzugte Ausführungsform der erfindungsgemäßen Zusammensetzung ist dadurch gekennzeichnet, dass ein polymeres Guanidin Biozid vorgesehen ist, welches ein Alkylendiamin und das Oxyalkylendiamin im Molverhältnis zwischen 4:1 und 1:4 enthält. Die Aminogruppen des Alkylendiamins und/oder des Oxyalkylendiamins sind bevorzugt endständig, wobei zur Herstellung des polymeren Guanidin Biozid als Alkylendiamin in erster Linie eine Verbindung der allgemeinen Formel NH₂(CH₂)ₙNH₂ vorgesehen ist, in welcher eine ganze Zahl zwischen 2 und 10, insbesondere 6, ist. Zur Herstellung des polymeren Guanidin Biozids kann als Oxyalkylendiamin eine Verbindung der allgemeinen Formel NH₂[(CH₂)₂O)]ₘ(CH₂)₂NH₂ vorgesehen werden, in welcher eine ganze Zahl zwischen 2 und 5, insbesondere 2, ist.

Bevorzugte polymere Guanidin Biozide können ausgewählt sein aus Poly(hexamethylenguanidin; Poly[2-(2-ethoxy)-ethoxyethyl)guanidin; Polytriethylenglykolguanidin; Polyethylenglykolguanidin; Polyoxypropylenguanidin; Polyoxyethylenguanidin.

Insbesondere bevorzugt ist das polymere Guanidin Biozid ein Polyalkylenguanidin, insbesondere ein Polyoxyalkylenguanidin. "Alkylen" kann ein C1-C8 Alkyl, bevorzugst ein C2-C6 Alkyl wie Ethyl, Propyl, Butyl, Methylpropyl, Pentyl sein, verzweigt oder unverzweigt. Dies ist in allen Verwendungen des Begriffs "Alkylen" oder "Alkyl" hierin bevorzugt.

Weitere bevorzugte polymeren Guanidine, die sich ausgezeichnet für die vorliegende Erfindung eignen, sind in WO 2014/113835 A1 und WO 2016/015081 A1 beschrieben. Derartige polymere Guanidine (auch "Polyguanidin" bezeichnet) können der Formel (I), (II) oder (III) entsprechen 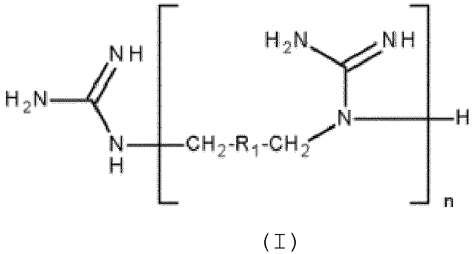 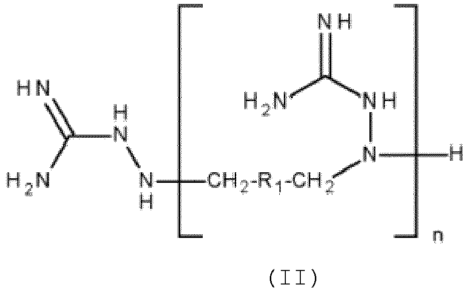 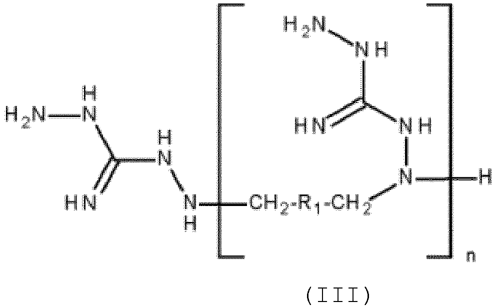 worin R₁ entweder für ein aromatisches Ringsystem mit zumindest einem aromatischen Ring, das gegebenenfalls ein oder mehrere Heteroatome, ausgewählt aus 0, N und S enthält und das gegebenenfalls mit einer oder zwei Vinylgruppen substituiert ist, oder für Ethylen steht, oder das eine durch Ringschluss unter Eliminierung eines Guanidins erhaltene zyklische Struktur aufweist. Beispiele für R1 sind Benzene (vorzugsweise para- oder meta-stellig eingebunden), Pyridin (vorzugsweise and den beiden C-Atomen benachbart zum N eingebunden), Divenylbenzol, Biphenyl (vorzugsweise jeweils beide Benzene para-stellig eingebunden), 1,3-Bis((E)-2-vinyl)benzol, Furan, Pyrrol, Thiophen, Fluoren, Ethylen (vorzugsweise cis-stellig eingebunden). Derartige polymere Guanidine sind in WO 2016/015081 A1 beschrieben.

Polymere Guanidine können auch die Formel (IV) enthalten 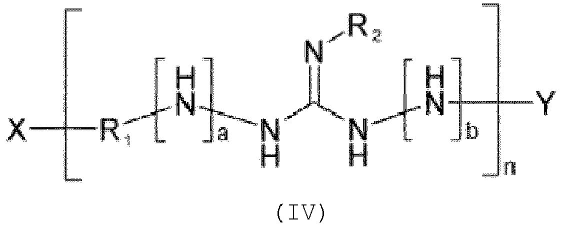 worin
X aus -NH₂, Aminoguanidino und 1 ,3-Diaminoguanidino ausgewählt ist;
Y aus -H und -R₁-NH₂ ausgewählt ist; oder X und Y zusammen für eine chemische Bindung stehen, um eine zyklische Struktur zu ergeben;
R₁ aus zweiwertigen organischen Resten mit 2 bis 20 Kohlenstoffatomen ausgewählt ist, in denen gegebenenfalls ein oder mehrere Kohlenstoffatome durch O oder N ersetzt sind; a und b jeweils 0 oder 1 sind,
wobei a+b ungleich 2 ist, wenn keine 1,3-Diaminoguanidin-Einheiten enthalten sind;
R₂ aus -H und -NH₂ ausgewählt ist,
wobei R₂ -NH₂ ist, wenn a+b=0 ist,
R₂ -H oder -NH₂ ist, wenn a+b=1 ist und
R₂ -H ist, wenn a+b=2 ist; und n größer gleich 2 ist;
oder ein Salz davon. Derartige poylmere Guanidine sind in WO 2014/113835 A1 beschrieben.

In den Formeln (I) bis (IV) n entspricht einer Vielzahl, z.B. um die oben genannten Molmassen des Polymers zu erreichen. Beispielsweise kann n 3 bis 200 betragen.

Vorzugsweise ist das Guanidin ein Guanidinium Salz, vorzugsweise ausgewählt aus einem Halogenid, vorzugsweise einem Chlorid; Phosphat, vorzugsweise einem Dihydrogenphosphat; Karbonat; Nitrat; Sorbat; Acetat, vorzugsweise Hydroacetat; Gluconat; Zitrat; Silikat; etc. Dies betrifft auch die polymeren Guanidine. Das erfindungsgemäße polymere Guanidin kann ein Polyguanidinsalz sein. Diese Verbindungen mit Salzbildungspartnern (Gegenionen), wie z.B. Halogenid, Phosphat, etc., sind in AT 411060 B beschrieben.

Vorzugsweise ist das mittlere Molekulargewicht des polymeren Guanidin Biozids 200 Da bis 10000 Da, vorzugsweise 500 Da bis 3000 Da. Z.B. kann das polymere Guanidin Biozid mit mindestens 3 Guanidinresten vorgesehen sein. Die Polymere mit diesen Größen können durch Nanofiltration durch Membranen erhalten werden. Zur Nanofiltration können Filter mit entsprechenden Porengrößen zur Durchlässigkeit der gewünschten Molmassen verwendet werden. Filtrate (zur Entfernung größerer Polymere) oder der Rückstand (zur Entfernung der kleineren Polymere) können weiter verwendet werden um das gewünschte Polymer zu erhalten. Die gewünschten vorzugsweisen polymere Guanidin Biozide haben eine verbesserte Toxikologie. Insbesondere der Eliminationen von kurzkettigen Oligomeren und Monomeren, sowie der Elimination von den durch die Synthese eingeschleppten Rückständen der Diamine kommt dabei ein Hauptaugenmerk zu. Diese könnten sonst von der Vaginalschleimhaut resorbiert werden, was zu unerwünschten pharmakologischen Wirkungen führen würde. Das Hauptaugenmerk der Formulierung liegt dabei auf der Lokalanwendung und trivalenten Wirkung ohne unerwünschten Erscheinungen. Dementsprechend werden monomere Ausgangsstoffe (Guanidine) und Oligo- oder Polymere des Guanidin Biozids mit einem Molekulargewicht von 250 Da oder kleiner, oder auch von 400 Da oder kleiner, entfernt.

Das polymere Guanidin Biozid wird in einer ausreichenden Menge oder Konzentration vorgesehen um das Wachstum pathogener Bakterien (gam negative und/oder gram positive), die beim Geschlechtsverkehr auftreten, zu hemmen. Derartige Bakterien die erfindungsgemäß gehemmt werden sind z.B. S. aureus, E. Coli, P. aeruginosa.

Alternativ oder in Kombination kann das polymere Guanidin Biozid in einer ausreichenden Menge oder Konzentration vorgesehen werden, um das Wachstum pathogener Pilze, die beim Geschlechtsverkehr auftreten, zu hemmen. Derartige Pilze die erfindungsgemäß gehemmt werden sind z.B. C. albicans, S. cerevisiae.

Alternativ oder in Kombination kann das polymere Guanidin Biozid in einer ausreichenden Menge oder Konzentration vorgesehen werden, um die Übertragung und Infektion des empfangenden Übertragungspartners durch Viren, die beim Geschlechtsverkehr auftreten, zu hemmen. Die biozide Wirkung des polymeren Guanidin Biozids richtet sich gegen diese Pathogene, vordergründig die Bakterien. Der Wirt bzw. der Geschlechtsverkehrspartner (insbesondere Menschen) soll möglichst durch das polymere Guanidin Biozid unbeeinträchtigt sein. Menschen sind die bevorzugten zu behandelnden Anwender der erfindungsgemäßen Zusammensetzung.

Die erfindungsgemäße Zusammensetzung kann weiters ein Alkylphenoxypolyethoxyethanol Spermizid, wie Nonylphenoxpoly(ethylenoxy)-ethanol (Nonoxynol-9), enthalten. Dies ist insbesondere für die Anwendung als "liquid condom", also als flüssiges, fluides oder bei Anwendung fluidisierbares (daher "liquid") Mittel zur Empfängnisverhütung zusätzlich zur Verminderung von Krankheitsübertragungen bevorzugt. Bevorzugte Spermizide die erfindungsgemäß eingesetzt werden können sind Nonoxynol-9, Octoxynol-9, Dodecaethylenglycolmonolaurat, Laureth 10S und Methoxypolyoxyethylenglycol 550 Laurat.

Bei besonderer Anwendung, nämlich bei der Anwendung von Homosexuellen, wird dieses Spermizid nicht benötigt. Stattdessen wird hierbei ein Lokalanästhetikum, wie Lidocain, verwendet um einen vorzeitigen Höhepunkt beim Geschlechtsverkehr, insbesondere beim Mann, zu verzögern. Ein derartiges Lokalanästhetikum kann auch bei einem heterosexuellen Geschlechtsverkehr verwendet werden, ist hier aber weniger bevorzugt aufgrund der Wirkung auf die Frau und es kann hier auch auf das Lokalanästhetikum verzichtet werden. Weiter Lokalanästhetika können vorzugsweise Benzocain, Dibucain, Benzylalkohol, Campher, Resorcin, Menthol und Diphenylhydraminhydrochlorid und dergleichen einschließen, sind aber nicht darauf beschränkt.

Alkylphenoxypolyethoxyethanol Spermizide wie Nonoxynol-9 sind bekannt und z.B. in der US 5,512,289 beschrieben. Wie einleitend erwähnt stellt die Kombination eines Alkylphenoxypolyethoxyethanol Spermizids mit einer wässrigen polymeren Gelmatrix Lösungsprobleme dar, wobei die US 5,512,289 vorschlug oberflächenaktive Substanzen, wie Polysorbat oder hohe PEG Mengen, einzusetzen. Erfindungsgemäß kann auf diese verzichtet werden, da überraschenderweise festgestellt wurde, dass das Alkylphenoxypolyethoxyethanol und das polymere Guanidin Biozid gegenseitige lösungsvermittelnde Rollen ausüben und aufgrund dieser synergistischen Wirkung besonders für eine Zusammensetzung für den Geschlechtsverkehr geeignet sind. Beide Substanzen (das polymere Guanidin Biozid und der Alkylphenoxypolyethoxyethanol) liegen erfindungsgemäß in Lösungen vor oder sind lösbar, und zwar vollständig ohne Phasentrennung oder Bildung einer trüben Emulsion aufgrund dieser chemischen Wechselwirkung.

Die erfindungsgemäße Zusammensetzung ist insbesondere ein Gleitgel oder Gleitmittel für den Geschlechtsverkehr.

Diese Gleitgele oder Gleitmittel haben eine erhöhte Viskosität, die durch ein Verdickungsmittel erreicht wird. Die Viskosität kann 1 Pas oder mehr betragen, insbesondere bevorzugt 1 Pas bis 900 Pas, speziell bevorzugt 7 Pas bis 500 Pas, insbesondere bevorzugt 35 Pas bis 150 Pas. Die Viskosität wird bei 36°C und Atmosphärendruck (1bar) bestimmt.

Vorzugsweise ist das Verdickungsmittel ein Gelbildner. Es kann z.B. ausgewählt sein aus Hydroxyalkylcellulose, Cellulose, Hydroxyethylcellulose, Hydroxypropylcellulose, Hydroxypropylmethylcellulose. Das Verdickungsmittel bei Zäpfchen kann ein Suppositorium-Trägermaterial sein, vorzugsweise ausgewählt wäre Hartfett (Adeps Neutralis), einer Glycerol-Gelatine-Mischung oder löslichem PEG, z.B. PEG 400/4000, wobei PEG weniger bevorzugt ist bzw. es nicht in der erfindungsgemäßen Zusammensetzung ist aufgrund der Wirkung auf Membranen und die Erhöhung ihrer Permeabilität.

Das Verdickungsmittel kann auch filmbildende Eigenschaften haben. Damit verbunden oder unabhängig vom Verdickungsmittel kann die Zusammensetzung vorzugsweise auch einen oder mehrere filmbildende Polymere, Gummi, Chitosane oder dergleichen enthalten, die z.B. von wasserlöslichen Cellulosen abgeleitet sind. Vorzugsweise sind solche von Cellulose abgeleiteten Polymere Hydroxyalkylcellulosepolymere. Bevorzugter ist das Hydroxyalkylcellulose-Polymer ausgewählt aus der folgenden Gruppe: Hydroxyethylcellulose, Carboxymethylcellulose, Hydroxypropylcellulose und Hydroxypropylmethylcellulose und dergleichen. Am meisten bevorzugt ist das Hydroxyalkylcellulose-Polymer Hydroxypropylcellulose. Alkyl kann wie oben zum Polymer definiert sein (C1-C8 Alykl, inkl. bevorzugte Ausführungsformen).

Die Zusammensetzung enthält vorzugsweise 0,05% bis 1% gew.-% polymeres Guanidin Biozid, insbesondere bevorzugt 0,1% bis 2%, speziell bevorzugt 0,4% bis 3% (alle gew.-%).

Die Zusammensetzung enthält vorzugsweise 0,05% bis 8 % (gew.%) Alkylphenoxypolyethoxyethanol Spermizid, insbesondere bevorzugt 0,1% bis 6%, speziell bevorzugt 0,3% bis 2% (alle gew.-%) .

Die Zusammensetzung enthält vorzugsweise 1% bis 95 % (gew.-%) Verdickungsmittel. Im Fall von (viskosen) Flüssigkeiten und Gelen enthält die Zusammensetzung vorzugsweise 1% bis 30% Verdickungsmittel, welche ein Gelbildner sein kann. Vorzugsweise ist der Bereich 1,5% bis 20%, insbesondere bevorzugt 2% bis 6% (alle gew.-%). Im Fall von Suppositorien ist die Menge Verdickungsmittel vorzugsweise 50% bis 95%, insbesondere bevorzugt 80% bis 93%.

Die Zusammensetzung kann in einer Einmaldarreichungsform vorgesehen werden, z.B. in einer Menge von 1ml bis 10ml, vorzugsweise 2ml bis 5ml. Eine Einmaldarreichungsform ist z.B. eine Einmalpipette. Eine Einmalpipette kann eine Kunststoffpipette sein, deren Kopf vor der Anwendung abzudrehen ist.

Die Zusammensetzung kann weiters ein Feuchthaltemittel enthalten, z.B. Propylenglycol oder Glycerin. Ein Feuchthaltemittel kann in einer Konzentration von 1% bis 30%, vorzugsweise 3% bis 18% (alle gew.-%) vorgesehen werden, insbesondere in einer flüssigen oder Gelzusammensetzung.

Vorzugsweise ist die Zusammensetzung eine homogene Zusammensetzung (keine unterschiedlichen Phasen) die als solches vaginal verabreicht werden kann. Sie benötigt insbesondere keine festen Träger wie einen Schwamm und wird ohne solchen festen Träger, der auch in der Anwendung fest bleibt (im Gegensatz zum Suppositorium), eingesetzt.

Die Zusammensetzung kann weiters Säuerungsmittel wie Acidum citricum oder mit zusätzlicher antioxidativer Wirkung, wie Acidum Ascorbicum enthalten.

Vorzugsweise hat die Zusammensetzung einen pH im Bereich von 4,5 bis 8. Insbesondere bevorzugt von 5 bis 7. Der pH kann durch herkömmliche Säuren und Basen eingestellt werden und ist vorzugsweise gepuffert, d.h. eine Säure oder Base sollte eine schwache Säure oder Base sein. Vorzugsweide sind Säuren ausgewählt aus Kohlensäure, Zitronensäure, Phosphorsäure, Essigsäure, Apfelsäure, Salzsäure, HEPES, etc..

Vorzugsweise werden nur die hiergenannten Substanzen verwendet und insbesondere keine (anderen) Substanzen, welche die Ephithelschicht Passage erhöhen, wie PEG oder Polysorbat. Lösungsvermittler aus der Gruppe der polyethoxylierten nichtionischen Verbindungen (dazu zählen ebenfalls Polyethylenglykol (PEG) oder Polysorbat (Tween)) werden ebenfalls vorzugsweise vermieden. Um nicht einfach die geschützte Erfindung gemäß dieser Ausführungsformen zu umgehen, können allerdings geringe oder unbedeutende Mengen dieser unerwünschten Substanzen vorhanden sein, z.B. maximal 1% oder maximal 0,5% (alle gew.-%).

Weitere unerwünschte Substanzen sind oxidierenden Substanzen wie Povidon-Iod, diese werden entweder komplett vermieden oder sind nur in geringen Mengen vorhanden, z.B. maximal 0,5% oder maximal 0,1% (alle gew.-%).

Ebenso zählen oberflächenaktive Substanz wie Benzalkonium Chlorid zu unerwünschten Substanzen, da sie die Durchlässigkeit der vaginalen Schleimhaut für Krankheitserreger erhöhen können. Diese Substanzen sind bevorzugt zu maximal 1%, speziell bevorzugt maximal 0,1% vorhanden (alle gew.-%). Nur um klarzustellen, die erfindungsgemäßen Wirkstoffe wie das polymere Guanidin Biozid oder das Spermizid oder das Lokalanästhetikum werden nicht zu den unerwünschten Stoffen gezählt.

Die Zusammensetzung kann auch pharmazeutische Trägerstoffe, Bindemittel, vorzugsweise polymere Bindemittel und/oder Zusatzstoffe enthalten. Der Begriff "Trägerstoff" bezieht sich auf ein Verdünnungsmittel, z.B. Wasser, Saline, Bindemittel oder Medium, mit dem die Zusammensetzung verabreicht werden kann. Bei einer festen oder flüssigen Zusammensetzung können die Trägerstoffe oder Zusatzstoffe in der pharmazeutischen Zusammensetzung SiO₂, TiO₂, ein Bindemittel wie etwa mikrokristalline Cellulose, Polyvinylpyrrolidon (Polyvidon oder Povidon), Tragantgummi, Gelatine, Stärke, Laktose oder Laktosemonohydrat, Alginsäure, Maisstärke und Ähnliches umfassen; ein Gleitmittel oder Tensid wie etwa Magnesiumstearat oder Natriumlaurylsulfat; ein Fließregulierungsmittel wie etwa kolloidales Silikondioxid.

Eine besonders bevorzugte Zusammensetzung gemäß der Erfindung enthält folgendes:

| | |
|---|---|
| polymeres Guanidin Biozid: | 0,05% bis 10%, |
| Feuchthaltemittel: | 5% bis 15%, |
| Alkylphenoxypolyethoxyethanol Spermizid: | 0,05% bis 10%, |
| Verdickungsmittel: | 1% bis 10%, |

alle % in gew.-%.

Die Zusammensetzung kann weiters enthalten:

| | |
|---|---|
| Acidum Ascorbicum und/oder Acidum Citricum: | 0% bis 3%, |
| Natrium Hydroxid: | 0% bis 2%, |
| Zitronensäure: | 0% bis 4%, |
| Aqua purificata: | Restauf auf 100%, |

alle % in gew.-%.

Statt oder zusätzlich zu Natriumhydroxid und Zitronensäure kann die Zusammensetzung auch andere Pufferkomponenten beinhalten, wie oben beschrieben, insbesondere um den pH Wert wie oben beschrieben einzustellen.

Die vorliegende Erfindung betrifft weiters eine Verwendung der erfindungsgemäßen Zusammensetzung gemäß einer beliebigen Ausführungsform zur vaginalen antibakteriellen, antimykotischen und/oder antiviralen Desinfektion. Ebenso betrifft die Erfindung die Verwendung derselben als Gleitmittel während eines Geschlechtsaktes sowie beide Funktionen in Kombination. Da Nonoxinol optional ist (z.B. als reines Gleitmittel ohne empfängnisverhütende Wirkung) betrifft die Erfindung auch die Verwendung einer chemischen Zusammensetzung enthaltend mindestens ein polymeres Guanidin Biozid in wässriger Lösung, und mindestens ein Verdickungsmittel zur vaginalen antibakteriellen, antimykotischen und/oder antiviralen Desinfektion während eines Geschlechtsaktes bzw. ein Verfahren zur Verhinderung einer Übertragung einer Geschlechtskrankheit während einem Geschlechtsakt durch Einbringung einer Zusammensetzung enthaltend mindestens ein polymeres Guanidin Biozid in wässriger Lösung, und mindestens ein Verdickungsmittel in die am Geschlechtsakt beteiligten Geschlechtsorgane, insbesondere Körperöffnungen. Vorzugsweise ist die Zusammensetzung oder einer ihrer Bestandteile oder Anwendung wie oben und unten definiert.

Die Erfindung betrifft weiters ein Verfahren zur Verhinderung einer Ansteckung einer sexuell übertragbaren Krankheit oder zur Empfängnisverhütung während eines Geschlechtsaktes, umfassend der Einbringung einer Zusammensetzung nach einer beliebigen Ausführungsform der Erfindung in einen Vaginalkanal zum Geschlechtsakt.

Ein mechanischer Schutz, wie ein Schwamm oder eine Barriere, durch feste Gegenstände in der Vagina sind nicht erforderlich und können bei der erfindungsgemäßen Anwendung unterbleiben.

Die erfindungsgemäße Zusammensetzung ist vorzugsweise ein Gleitgel im Sinne einer viskosen Flüssigkeit, worin die Wirkstoffe gelöst sind. Ein Gleitgel hat den Vorteil eines schmerzfreien und reibungsreduzierten Geschlechtsverkehrs. Die bei Frauen vor allem in der Postmenopause auftretende Trockenheit der Vaginal-Schleimhaut wird durch den Gelcharakter der Formulierung entgegen gewirkt, wodurch keine Schmerzen beim Sexualakt auftreten oder dessen Risiko reduziert werden.

Weiters kann ein Lokalanästhetikum wie oben beschrieben verwendet werden, um einer frühzeitigen Ejakulation des Mannes entgegen zu wirken.

Die erfindungsgemäße Zusammensetzung kann sowohl für heterosexuelle als auch für homosexuelle Männer angewendet werden. Statt einer Übertragung von Krankheitserregern in einer Vagina kann diese Übertragung auch in anderen Körperöffnungen verhindert werden, z.B. im Anus. Der Vorteil liegt beim gleichgeschlechtlichen Geschlechtsverkehr ebenso im Gelcharakter der Anwendungsform, der einen reibungsreduzierten Geschlechtsverkehr ermöglicht und der Unterbrechung der Übertragung von Geschlechtskrankheiten. Durch den Gelcharakter der Zusammensetzung werden Mikroläsionen der Analschleimhaut massiv reduziert, ein häufig auftretendes Problem bei der Anwendung von herkömmlichen Kondomen, die in weiterer Folge zur Ruptur neigen. Durch die Vermeidung von Mikroläsionen an der Schleimhaut wird die Ansteckungsgefahr mit pathogenen Keimen bereits deutlich reduziert.

Die erfindungsgemäße Zusammensetzung kann eingesetzt werden oder für eine Anwendung vorgesehen sein, um eine Übertragung von Pathogenen zu reduzieren. Derartige Pathogene können Bakterien, Pilze oder Viren sein. Bakterien, deren Übertragung erfindungsgemäß gehemmt wird, sind z.B. S. aureus, E. Coli, P. aeruginosa. Pilze, deren Übertragung erfindungsgemäß gehemmt wird, sind z.B. C. albicans, S. cerevisiae. Die antivirale Wirkung von polymeren Guanidinen und Biguanidinen ist in der Literatur vielfach beschrieben (z.B. Klein et al. Journal of General Virology (2000), 81, 895-901).

Insbesondere gegen unbehüllte Viren zeigt das polymere Guanidin starke virucide Aktivität. In der Literatur ist die antivirale Aktivität gegen klassische Viren beschrieben, die durch Geschlechtsverkehr übertragen werden und gegen die es sich zu schützen gilt, Teils durch Test mit dem strukturähnlichen polymeren Guanidinderivat Polyhexamethylenbiguanid (PHMB), siehe Romanowski et al. (JAMA Ophthalmol. 2013 Apr;131(4):495-8), Gentile et al. (BMC Clinical Pathology201212:17), Valluri et al. (Cornea. 1997; 16 (5) :556-559), Passic et al. (Biomed Pharmacother. 2010;64(10):723-732). Die erfindungsgemäße Zusammensetzung kann durch die Wirkstoffe und durch die Gel/Säure/Polymermischung die Übertragung von Viren von den Virenträger auf die Zellen der Kontaktperson reduzieren oder verhindern. Viren, deren Übertragung reduziert oder verhindert wird sind beispeilsweise HPV (Humane Papillomviren), HSV (Herpes-simplex-Virus) oder HIV (Humanes Immundefizienz-Virus).

Die Erfindung kann eine gezielte Verhinderung der Übertragung einer Infektion mit diesen Pathogenen betreffen, z.B. nach Diagnose an einem der Geschlechtsverkehrspartner, oder eine prophylaktische Anwendung, z.B. um eine allfällige Übertragung zu verhindern.

Die vorliegende Erfindung wird weiter durch die folgenden Figuren und Beispiele beispielhaft beschrieben.

Die **Figuren** zeigen die hemmende Wirkung der erfindungsgemäßen Zusammensetzung und reinem Akacid in unterschiedlichen Wirkstoffkonzentrationen gegen diverse Pathogene, nämlich gegen MRSA1 (Fig. 1), EK4 (Fig. 2), Strepto 8(Fig. 3), E. Coli 13 (Fig. 4), KL 37, (Fig. 5), PS23 (Fig. 6), Asp. Fum. 45 (Fig. 7), Asp. Faec. 46 (Fig. 8), C. albicans (Fig. 9), C. krusei (Fig. 10), jeweils zusammen mit Positiv- (PC) und Negativkontrollen (NC).

### Beispiel 1: Herstellung einer Polyguanidinformulierung

Grundlage der Formulierungen sind biozide Wirkstoffe der Gruppe der Polyguanidine (Akacid, beschrieben in Kratzer et al., Antibiotika Monitor 1/2/2006; Buxbaum et al., Journal of Antimicrobial Chemotherapy (2006) 58, 193-197; WO 01/85676 A1; WO 2006/047800 A1; WO 2008/080184 A2; WO 2013/064161 A1), in diversen Formulierungen wie Akacid Plus (3:1-Mischung aus Polyhexamethylen-guanidinium-chlorid) und Poly-[2-(-ethoxy)ethoxyethyl)-guanidinium-chlorid). Diese bioziden polymeren Guianidinverbindungen zeigen ein ausgezeichnetes antimikrobielles Profil und sind zudem in der Lage stabile Formulierungen mit schwer mischbaren Substanzen ohne Lösungsvermittler aus der Gruppe der polyethoxylierten nichtionischen Verbindungen zu vermitteln.

Akacid wurde in einem modifizierten Verfahren hergestellt indem die Ausgangssubstanzen Triethylenglykol mit dem Hexamethylendiamin und Guanidin hydrochlorid in einem 1-Schritt Synthese Verfahren hergestellt wurden und anschließend durch die Filtration durch drei NF Membranen von niedermolekularen und hochmolekularen Monomeren zu befreien, sodass eine monomere bereinigte Akacid Plus Fraktion mit besseren pharmakologischen Eigenschaften entsteht. Das mittlere Molekulargewicht von diesem Akacid Plus (oder "X-Cid") liegt bei etwa 1000 Dalton (hauptsächlich 500 bis 3000 Dalton). Die Zusammensetzung wurde als wässrige Lösung zur weiteren Formulierung bereitgestellt.

Wirkstoffmengen werden in g oder % angegeben. Alle %-Angaben betreffen Gewichts-%, sofern nichts anderes angegeben ist.

### Beispiel 2: Formulierung "Liquid Condom Female"

Die Formulierung des "Liquid Condom Female" besteht aus einem Hydroxyaethylcellulose-hältigen Gel. Die Hydroxyaethylcellulose wird mit Aqua Purificata 5 Minuten gequollen, bis ein dünnflüssiges Gel entstanden ist.

In diese Gelgrundlage wird eine Zitronensäurelösung eingearbeitet, die mittels einer Kaliumhydroxid Lösung auf einen PH Wert von 5,5-6,2 nach Beimengung sämtlicher weiterer Substanzen eingestellt wird.

Die zwei aktiven Wirkstoffe Akacid Plus 1000 (in einer Konzentration von 0,2-1%) und der spermizide Wirkstoff 9-Nonoxinol (in einer Konzentration von 5%) oder andere Wirkstoffe die eine Empfängnisverhütung begünstigen wie Milchsäure, Zitronensäure, Chinin, Granatapfelkernextrakt, Honig oder zerriebene Akaziensprossen werden in die fertige Gelgrundlage eingearbeitet. Die Darreichung des Gels erfolgt über 1,8 ml Einmal-Plastikpipetten, deren Kopf vor der Anwendung abzudrehen ist. Die Gesamtmenge ist mindestens 1-3 Minuten vor dem Geschlechtsverkehr intravaginal einzuspritzen. Die Verteilung des Gels wird dabei durch den Geschlechtsakt selbst bedingt.

Rezeptur I "liquid condom female":

| | |
|---|---|
| Akacid Plus 1000 50% | 0,8 g (also 0,4 g Akacid) |
| Acidum Citricum | 1,5 g |
| Propylenglycolum | 10,0 g |
| Nonoxinol-9 | 5,0 g |
| Hydroxyaethylcellulose | 2,0 g |
| Natrium Hydroxid | 0,75 g |
| Aqua purificata | 79,95 g |
| | 100,0 g |

### Beispiel 3: Formulierung "Liquid Condom Male"

Die Formulierung des "Liquid Condom Male" ist ein Hydroxyaethylcellulose haltiges Gel. Die Hydroxyaethylcellulose wird mit Aqua Purificata 5 min gequollen bis ein zähflüssiges Gel unter Reiben entstanden ist. Die zwei aktiven Wirkstoffe Akacid Plus 1000 (z.B. in einer Konzentration von 0,2%) und das Lokalanästhetikum Lidocainum hydrochloricum (in einer Konzentration von 0,4 %) werden in die bestehende Salbengrundlage eingearbeitet. Das Lokalanästhetikum ist zur Desensibilisierung enthalten um einer frühzeitigen Ejakulation entgegen zu wirken. Der pH Wert ist dabei neutral bis schwach basisch. Die Formulierung hat keine kontrazeptive Wirkung und dient primär entgegen der Ansteckung des Mannes durch einen an sonst ungeschützten Geschlechtsverkehr.

### Rezeptur II "liquid condom male":

| | |
|---|---|
| Akacid Plus 1000 50% | 0,6 g (also 0,3 g Akacid) |
| Propylenglycolum | 3,0 g |
| Hydroxyaethylcellulose | 3,0 g |
| Lidocain Hydrochloricum | 0,4 g |
| Aqua purificata | 93, 0 g |
| | 100,0 g |

### Beispiel 4: Formulierung "Liquid Condom Suppository"

Die eingewogene Akacid Plus 1000 Menge wird in die aufgeschmolzene Adeps Neutralis Lösung gemeinsam mit dem Nonoxinol-9 eingearbeitet und nach Abkühlung auf ca. 40 -45 C° in Zäpfchen Formen 2g gegossen und abgekühlt gelassen bis zum Erstarren.

### Rezeptur III "liquid condom suppository 1%" 10 Stk

| | |
|---|---|
| Akacid Plus 1000 50% | 0,4 g (also 0,2 g Akacid) |
| Adeps Neutralis | 19,0 g |
| Nonoxinol-9 | 1,6 g |
| | 21,0g |

### Beispiel 5: Vergleich der antibakteriellen Wirkung des "liquid condom" im Vergleich zu reinem Akacid

Die folgenden Zusammensetzungen wurden im Vergleich zueinander getestet, wobei nur die Akacid Konzentration (als Akacid Plus nach Beispiel 1) in verschiedenen Verdünnungen variiert wurde.
Zusammensetzung 1: "liquid condom" nach Beispiel 2 ("liquid condom female"); mit Nonoxinol-9
Zusammensetzung 2: "liquid condom" nach Beispiel 2 ("liquid condom female") ohne Nonoxinol-9 (stattdessen Wasser)
Zusammensetzung 3: Akacid Plus, Rest: Wasser
getestete Pathogene:
- MRSA 1 - Staphylococcus aureus
- EK 4 - Enterococcus faecium
- Strepto 8 - Streptococus pneumoniae
- E.coli 13 - Escherichia coli
- KL 37 - Klebsiella pneumonia
- PS 23 - Pseudomonas aeruginosa
- Asp. Fum. 45 - Aspergillus fumigatus
- Asp. Faec. 46 - Aspergillus fumigatus
- C. Alb. - Candida albicans
- C. Krusei 26 - Candida krusei

### Medien:

Für Bakterien: Müller Hinton Bouillon
Für Pilze: Sabouraud Bouillon

### Assay:

50µl des jeweiligen Mediums wurden in alle Vertiefungen von 96-Well-Platten gegeben, mit Ausnahme der Kontroll-Vertiefungen, in denen 100µl Medium pro Vertiefungen zugegeben wurden. Proben wurden präpariert und 100µl davon wurden in die Vertiefungen gegeben.

50 µl der Testzusammensetzungen wurden den Vertiefungen zugegebenen, wobei die Testzusammensetzungen unterschiedliche X-Cid-Konzentrationen aufwiesen, beginnend von 0,6%, jeweils 1:1 Verdünnungen mit den restlichen Zusammensetzungsbestandteilen, also 0,6%, 0,3%, 0,15%, 0,075%, 0,0375%, 0,0188%, 0,00938%, 0,00469%, 0,00234%, 0,00117% (gerundet, alle % in gew.-%).

Erregerproben wurden aufgetaut und im jeweiligen Medium verdünnt. 50µl der Erregerprobe wurden dann in alle Vertiefungen gegeben (außer Negativkontrolle). Die Inkubation erfolgte über Nacht bei 35°C. Am nächsten Tag wurden die Platten visuell ausgewertet und mit dem SpectraMax 190 Reader vermessen.

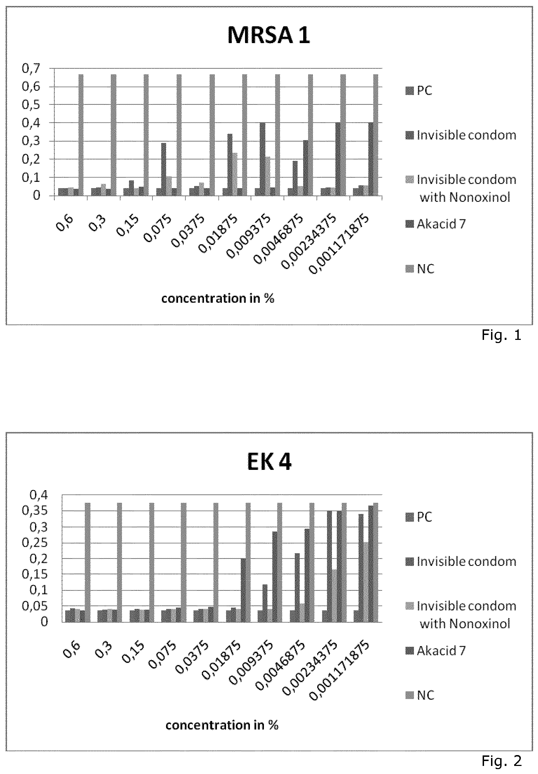

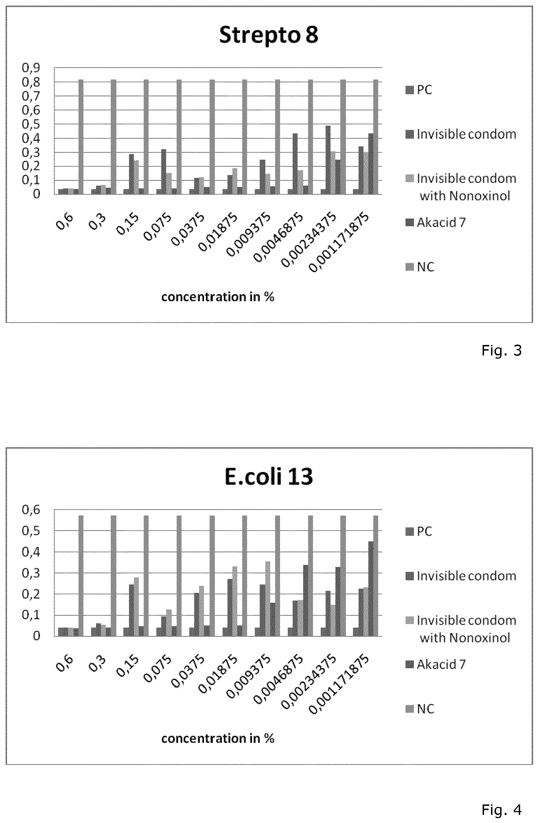

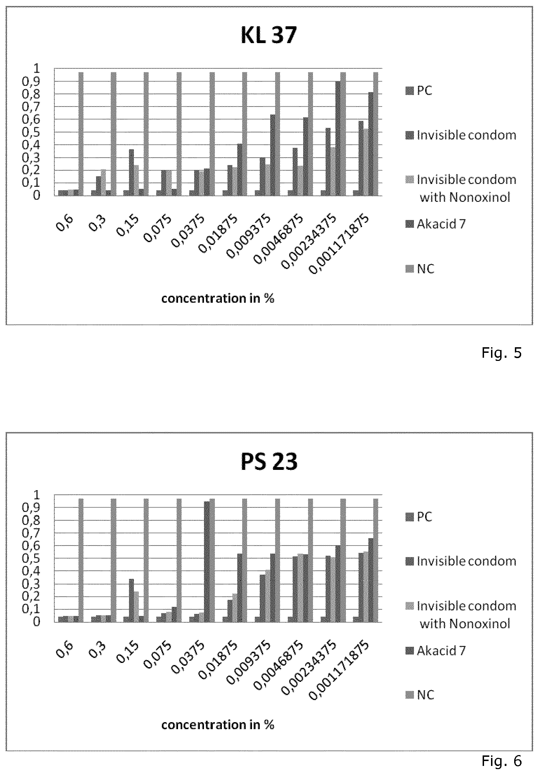

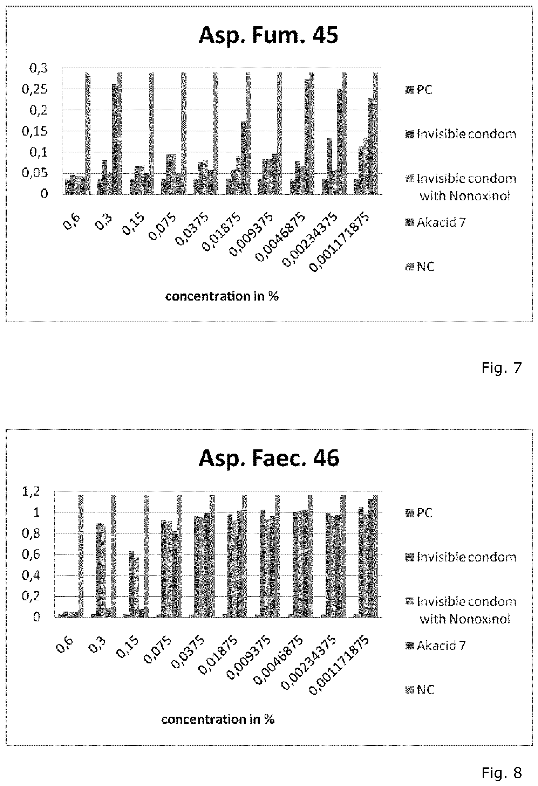

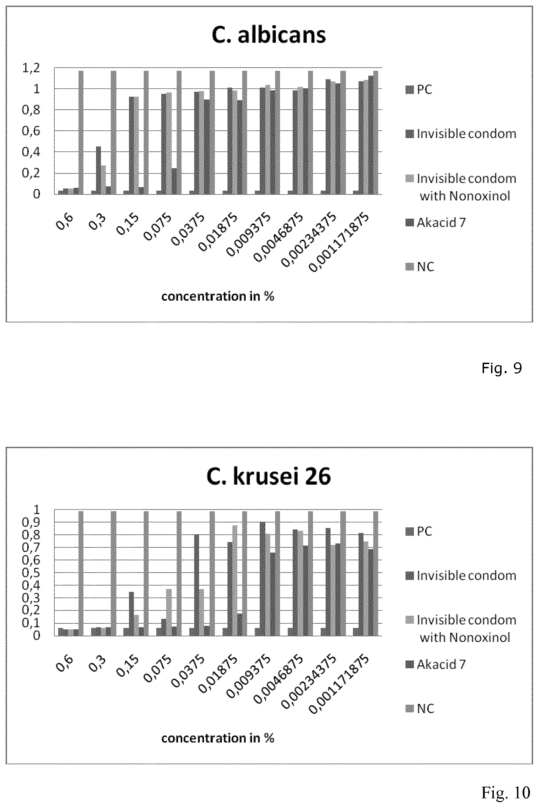

Die Ergebnisse der Messung mit dem SpectraMax190 Reader sind in den Figuren 1-10 gezeigt.

Offensichtlich gab es bei MRSA eine Aktivität des "liquid condoms" in allen Konzentrationen. Einzelne Ausreißer sind darauf zurückzuführen, dass möglicherweise ungleiche Stoffmengen mit der Pipette weitertransportiert werden. Das kann möglich sein, da die Substanz selbst sehr zähflüssig war und es daher manchmal schwierig war, exakte Mengen zu pipettieren.

Im "liquid condom" mit Nonoxinol war es dasselbe.

Für Akacid 7(=X-Cid Testsubstanz) konnte eine Aktivität bis zu einer Konzentration von 0,009375% beobachtet werden. Danach konnte keine Hemmung mehr erreicht werden.

Zu EK 4 konnte die Aktivität des "liquid condoms" bis zu einer Konzentration von 0,08175% beobachtet werden, während die Aktivität des "liquid condoms" in Kombination mit Nonoxinol bis zu einer Konzentration von 0,009375% beobachtet werden konnte.

Akacid 7 war bis zu einer Konzentration von 0,0375% aktiv. Überraschenderweise war somit das "liquid condom" bei gleicher Akacid Konzentration effektiver als reines Akazid. Dies kann nur auf synergistische Wirkungen der Zusammensetzung zurückzuführen sein.

Zu Streptokokkus 8 waren wiederum Schwankungen in der Sichtbarkeit der Aktivität vorhanden, so dass eine echte Hemmung nur in den ersten beiden Konzentrationen erreicht werden konnte.

### Für

Bei Akacid 7 konnte eine Aktivität gegen Streptococcus 8 bis zu einer Konzentration von 0,0046875% beobachtet werden.

Bei E.coli 13 waren wiederum Schwankungen in der Aktivität für das "liquid condom" sowie für das "liquid condom" in Kombination mit Nonoxinol zu beobachten. Für Akacid 7 wurde die Aktivität bis zu einer Konzentration von 0,01875% angegeben.

Zu KL 37 wurde die Aktivität des "liquid condoms" bis zu einer Konzentration von 0,009375% gegeben, während die Aktivität des "liquid condoms" in Kombination mit Nonoxinol bis zu einer Konzentration von 0,0046875% gegeben wurde. Für Akacid 7 konnte eine Aktivität bis zu einer Konzentration von 0,0375% beobachtet werden.

Zu PS 23 wurde die Aktivität für Akacid 7 bis zu einer Konzentration von 0,0375% festgestellt. Für das "liquid condom" und die Kombination von "liquid condoms" und Nonoxinol-Aktivität wurden bis zu einer Konzentration von 0,01875% eine Hemmung festgestellt.

Zu Aspergillus fumigatus 45 wurde die Aktivität des "liquid condoms" und die Kombination des "liquid condoms" mit Nonoxinol fast in allen Konzentrationen gegeben (bis 0,00118% - minimale getestete Konz.). Bei Akacid 7 wurde die Aktivität nur bis 0,009% erreicht. Zu Aspergillus fumigatus 46 wurde die Aktivität für das "liquid condom" sowie für die Kombination von "liquid condom" mit Nonoxinol bei 0.6% vollständig gegeben. Für Akacid 7 wurde die Aktivität für einen Konzentrationswert von 0,15% festgestellt. Bei Candida albicans war die Aktivität für das "liquid condom" sowie für das "liquid condom" in Kombination mit Nonoxinol bis 0,3% gegeben. Für Akacid 7 war die Hemmung bis zu einer Konzentration von 0,075% sichtbar.

Für Candida krusei 26 war die Hemmung für Akacid 7 die höchste im Vergleich zu "liquid condoms" und der Kombination von "liquid condom" mit Nonoxinol, und zwar mit einer Hemmung bis zu einer Konzentration von 0,08175% von Akacid. Für das "liquid condom" war eine Hemmung bis zu einer Konzentration von 0,075% sichtbar. Für "liquid condoms" in Kombination mit Nonoxinol war Hemmung bis zu einer Konzentration von 0,0375% gegeben.

Zusammenfassend wurde fast kein oder nur ein sehr geringer Unterschied zwischen dem "liquid condom" allein und "liquid condoms" mit Nonoxinol festgestellt. Akacid 7 zeigte jedoch in den meisten Fällen einen geringeren Hemmungseffekt im Vergleich zu den beiden "liquid condoms" Zusammensetzungen.

## Patentansprüche

1. Chemische Zusammensetzung enthaltend mindestens ein polymeres Guanidin Biozid und mindestens ein Alkylphenoxypolyethoxyethanol Spermizid oder ein Lokalanästhetikum, in wässriger Lösung, und mindestens ein Verdickungsmittel.

2. Zusammensetzung nach Anspruch 1, **dadurch gekennzeichnet, dass** das polymere Guanidin Biozid ein Polyalkylenguanidin, vorzugsweise ein Polyoxyalkylenguanidin, ist.

3. Zusammensetzung nach Anspruch 2, **dadurch gekennzeichnet, dass** das polymere Guanidin Biozid ausgewählt ist aus Poly(hexamethylenguanidin; Poly[2-(2-ethoxy)-ethoxyethyl)-guanidin; Polytriethylenglykolguanidin; Polyethylenglykolguanidin; Polyoxypropylenguanidin; Polyoxyethylenguanidin.

4. Zusammensetzung nach einem der Ansprüche 1 bis 3, **dadurch gekennzeichnet, dass** das Guanidin ein Guanidinium Salz ist, vorzugsweise ausgewählt aus einem Halogenid, vorzugsweise einem Chlorid; Phosphat, vorzugsweise einem Dihydrogenphosphat; Karbonat; Nitrat; Sorbat; Acetat, vorzugsweise hydroacetat; Gluconat; Zitrat; Silikat.

5. Zusammensetzung nach einem der Ansprüche 1 bis 4, **dadurch gekennzeichnet, dass** das mittlere Molekulargewicht des polymeren Guanidin Biozids 200 Da bis 10000 Da ist, vorzugsweise 500 Da bis 3000 Da.

6. Zusammensetzung nach einem der Ansprüche 1 bis 5, **dadurch gekennzeichnet, dass** das Alkylphenoxypolyethoxyethanol Spermizid Nonylphenoxpoly-(ethylenoxy)-ethanol (Nonoxynol-9) ist.

7. Zusammensetzung nach einem der Ansprüche 1 bis 6, **dadurch gekennzeichnet, dass** das Verdickungsmittel ein Gelbildner ist, vorzugsweise ausgewählt ist aus Hydroxyalkylcellulose, Celllose, Hydroxyethylcellulose, Hydroxypropylcellulose, Hydroxypropylmethylcellulose; oder ein Suppositorium-Trägermaterial ist, vorzugsweise ausgewählt aus Hartfett, einer Glycerol-Gelatine-Mischung oder löslichem PEG, z.B. PEG 400/4000.

8. Zusammensetzung nach einem der Ansprüche 1 bis 7, **dadurch gekennzeichnet, dass** die Zusammensetzung 0.05 bis 10 % (gew.-%) polymeres Guanidin Biozid umfasst.

9. Zusammensetzung nach einem der Ansprüche 1 bis 8, **dadurch gekennzeichnet, dass** die Zusammensetzung 0,05 bis 8 % (gew.-%) Alkylphenoxypolyethoxyethanol Spermizid umfasst.

10. Zusammensetzung nach einem der Ansprüche 1 bis 9, **dadurch gekennzeichnet, dass** die Zusammensetzung 1 bis 95 % (gew.-%) Verdickungsmittel umfasst.

11. Zusammensetzung nach einem der Ansprüche 1 bis 10, **dadurch gekennzeichnet, dass** die Zusammensetzung in einer eimaldarreichungsform in einer Menge von 1 ml bis 5 ml ist, vorzugsweise in einer Einmalpipette.

12. Zusammensetzung nach einem der Ansprüche 1 bis 11, **dadurch gekennzeichnet, dass** die Zusammensetzung ein Feuchthaltemittel, vorzugsweise Propylenglycol, enthält.

13. Zusammensetzung nach einem der Ansprüche 1 bis 12, **dadurch gekennzeichnet, dass** die Zusammensetzung folgendes enthält:
| | |
|---|---|
| polymeres Guanidin Biozid: | 0,05% bis 10%, |
| Feuchthaltemittel: | 5% bis 15%, |
| Alkylphenoxypolyethoxyethanol Spermizid: | 0,05% bis 10%, |
| Verdickungsmittel: | 1% bis 10%, |
alle % in gew.-%;
vorzusgwese weiters enthält:
| | |
|---|---|
| Acidum Ascorbicum: | 0% bis 3%, |
| Natrium Hydroxid: | 0% bis 2%, |
| Zitronensäure: | 0% bis 4%, |
| Aqua purificata: | Rest auf 100%, |
alle % in gew.-%.

14. Verwendung einer chemischen Zusammensetzung enthaltend mindestens ein polymeres Guanidin Biozid in wässriger Lösung, und mindestens ein Verdickungsmittel zur vaginalen antibakteriellen, antimykotischen und/oder antiviralen Desinfektion während eines Geschlechtsaktes.

15. Verfahren zur Verhinderung einer Ansteckung einer sexuell übertragbaren Krankheit oder zur Empfängnisverhütung während eines Geschlechtsaktes, umfassend der Einbringung einer Zusammensetzung nach einem der Ansprüche 1 bis 13 in einen Vaginalkanal zum Geschlechtsakt.
